# EUROPÄISCHE PATENTSCHRIFT

(11) **EP 2 367 582 B1**
(45) Veröffentlichungstag und Bekanntmachung des Hinweises auf die Patenterteilung: **25.10.2017**
(21) Anmeldenummer: 09765035.2
(22) Anmeldetag: 03.12.2009
(51) Int. Cl.: A61M 5/30

(54) **EINMALINJEKTOR MIT EINEM BIEGEELASTISCHEN METALLGEHÄUSE**
SINGLE-USE INJECTOR HAVING A FLEXURALLY ELASTIC METAL HOUSING
INJECTEUR À USAGE UNIQUE DOTÉ D'UN BOÎTIER MÉTALLIQUE ÉLASTIQUE EN FLEXION

(30) Priorität: 18.12.2008 DE 102008063519
(43) Veröffentlichungstag der Anmeldung: 28.09.2011
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2009/008612
(87) Internationale Veröffentlichungsnummer: WO 2010/069468

(56) Entgegenhaltungen:
- WO-A1-2005/044344
- DE-A1-102007 008 369
- GB-A- 805 184
- US-A- 3 094 989
- US-A- 3 557 784
- US-A1- 2005 020 984

## Beschreibung

Die Erfindung betrifft einen nadellosen Einmalinjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Kolbenbetätigungsstempel zwischen dem Federenergiespeicher und dem Kolben der Zylinder-Kolben-Einheit positioniert ist, wobei der Federenergiespeicher mindestens ein vorgespanntes Federelement umfasst, wobei der federbelastete Kolbenbetätigungsstempel über Stützstäbe oder Zughaken am Gehäuse abgestützt ist und wobei die zwischen einem einzelnen Stützstab oder Zughaken und dem Kolbenbetätigungsstempel gelegene Kontaktzone ein den jeweiligen Stützstab oder Zughaken nach außen drängendes Keilgetriebepaar darstellt.

Aus der DE 10 2007 031 630 A1 ist u.a. ein derartiger Injektor bekannt. Mit Ausnahme der mechanischen Feder des Federenergiespeichers sind nahezu alle Bauteile des Injektors aufwendig aus Kunststoffen durch Spritzgießen gefertigt. Mechanisch hochbelastete Bauteile sind zusätzlich glasfaserverstärkt ausgeführt.

Die DE 10 2007 008 369 A1 offenbart einen Einweginjektor gemäß dem Oberbegriff des Anspruchs 1, mit einem Gehäuse, in dem oder an dem - jeweils zumindest bereichsweise - ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst und wobei zumindest ein Teil des Kolbenbetätigungsstempels zwischen dem Federenergiespeicher und dem Kolben der Zylinder-Kolben-Einheit positioniert ist. Der federbelastete Kolbenbetätigungsstempel weist mindestens einen Zugstab auf, der im Bereich seines hinteren Endes mindestens eine Abstützfläche hat. An der oder den Abstützflächen liegen am Gehäuse abgestützte Sperrelemente an, deren sperrende Lage durch ein in einer Sperrstellung positioniertes Auslöseelement gesichert ist und dass das Auslöselement eine Lösestellung hat, die ein Freigeben der Sperrelemente bewirkt.

Die WO 2005/044 344 A1 offenbart eine Vorrichtung für die Verabreichung eines injizierbaren Produkts, die ein Gehäuse, ein von dem Gehäuse aufgenommenes Produktbehältnis, eine in die Vortriebsrichtung auf den Kolben wirkende Kolbenstange und eine in die Vortriebsrichtung auf die Kolbenstange wirkende Feder umfasst. Das Produktbehältnis ist bewegbar gelagert und weist einen Kolben auf, der in eine Vortriebsrichtung bewegbar aufgenommen ist, um ein Produkt auszuschütten. Die Kolbenstange wird gegen die Kraft der Feder in einer Halteposition in einem Halteeingriff lösbar gehalten. Die Feder ragt in der Halteposition in das Behältnis hinein.

Der vorliegenden Erfindung liegt daher die Problemstellung zugrunde, einen modular aufgebauten Einmalinjektor zu entwickeln, der bei geringer Baugröße nur wenige Bauteile aufweist und bei einfacher Handhabung sowie einer kostengünstigen Herstellung eine sichere Lagerung und Funktion gewährleistet.

Diese Problemstellung wird mit den Merkmalen des Hauptanspruchs gelöst. Dazu besteht das Gehäuse des Injektors aus einem dünnwandigen Blechteil. Das Blechteil weist mindestens zwei Schenkel auf. Die Schenkel weisen an den freien Enden - als Aufnahme des Zylinders der Zylinder-Kolben-Einheit - jeweils ein abgewinkeltes Halteelement oder eine Ausnehmung auf. Das Blechteil weist mindestens zwei Druckstäbe oder mindestens zwei Zughaken auf, deren freie Enden zur Ausbildung eines Abstützabschnitts für den Kolbenbetätigungsstempel jeweils winkelförmig abgebogen sind. Die Auslöseeinheit umfasst mindestens ein auf dem Gehäuse gleitfähig angeordnetes Auslöseelement, wobei das Auslöseelement mit Fenstern oder Längsnuten ausgebildet ist, die die Abstützabschnitte der Stützstäbe oder Abstützabschnitte der Zughaken nach dem Auslösen des Einmalinjektors aufnehmen.

Mit der Erfindung wird hier beispielsweise ein nadelfreier Einmalinjektor vorgestellt, dessen Kolbenbetätigungsstempel bei einem Auslösevorgang des Einmalinjektors freigegeben wird. Dazu wird zum Vorspannen und Halten des Federenergiespeichers der Kolbenbetätigungsstempel über mindestens einen am Gehäuse angeordneten oder im Gehäuse integrierten Stützstab oder Zughaken formschlüssig gehalten. Der oder die Stützstäbe bzw. Zughaken werden von einem das Gehäuse zumindest bereichsweise umgebenden Auslöseelement bis zum Gebrauch des Einmalinjektors in ihrer Sperrposition gehalten. Zum Auslösen des Injektors werden der oder die Stützstäbe bzw. Zughaken freigegeben, so dass sich der Kolbenbetätigungsstempel - unter der Wirkung des Federenergiespeichers - zumindest annähernd parallel zur Mittellinie des Einmalinjektors bewegen kann, um die im Zylinder der Zylinder-Kolben-Einheit vorhandene Injektionslösung über mindestens eine Düse auszustoßen.

Das Gehäuse ist dabei ein einfaches, dünnwandiges Blechteil, ggf. sogar nur ein Blechstreifen, das die mechanische oder pneumatische Feder des Federenergiespeichers zusammen mit einem Kolbenbetätigungsstempel und einer Zylinder-Kolben-Einheit unter Zusammenwirkung mit dem Auslöseelement lagert. Das gestanzte oder geschnittene, mehrfach gebogene Blechteil ist außerordentlich kostengünstig aus einem Eisenwerkstoff oder einem Buntmetall herstellbar. Ideal sind Werkstoffe, die eine hohe Elastizitätsgrenze, eine hohe Zugfestigkeit und ein hohes Streckgrenzenverhältnis aufweisen.

Weitere Einzelheiten der Erfindung ergeben sich aus den Unteransprüchen und den nachfolgenden Beschreibungen schematisch dargestellter Ausführungsbeispiele.
- Figur 1:: Einweginjektor mit zwei Stützstäben;
- Figur 2:: wie Figur 1, jedoch um 90 Winkelgrade geschwenkt;
- Figur 3:: Querschnitt zu Figur 2;
- Figur 4:: Längsschnitt des Blechstreifens;
- Figur 5:: oberer Bereich des Blechstreifens;
- Figur 6:: Einweginjektor bei einem Montagezwischenschritt;
- Figur 7:: oberer Gehäusebereich während der Montage;
- Figur 8:: Querschnitt zu Figur 7;
- Figur 9:: wie Figur 1, jedoch entsichert und betätigt;
Die zumindest annähernd parallel zueinander ausgerichteten Schenkel (220) sind an ihren freien Enden jeweils um 90 Winkelgrade nach innen abgewinkelt, um dort jeweils ein Halteelement (221) zu bilden. Die Halteelemente (221), die z.B. 1,5 bis 3 Millimeter lang sind, ragen aufeinander zu. Sie bilden eine Ebene, die parallel zur Stirnplatte (210) ausgerichtet ist.

Anstelle des hakenförmigen Halteelements (221) kann in jedem Schenkel (220) eine Ausnehmung vorgesehen sein, in die der Zylinder der Zylinder-Kolben-Einheit (100) mittels jeweils eines Zapfens eingehängt werden kann.

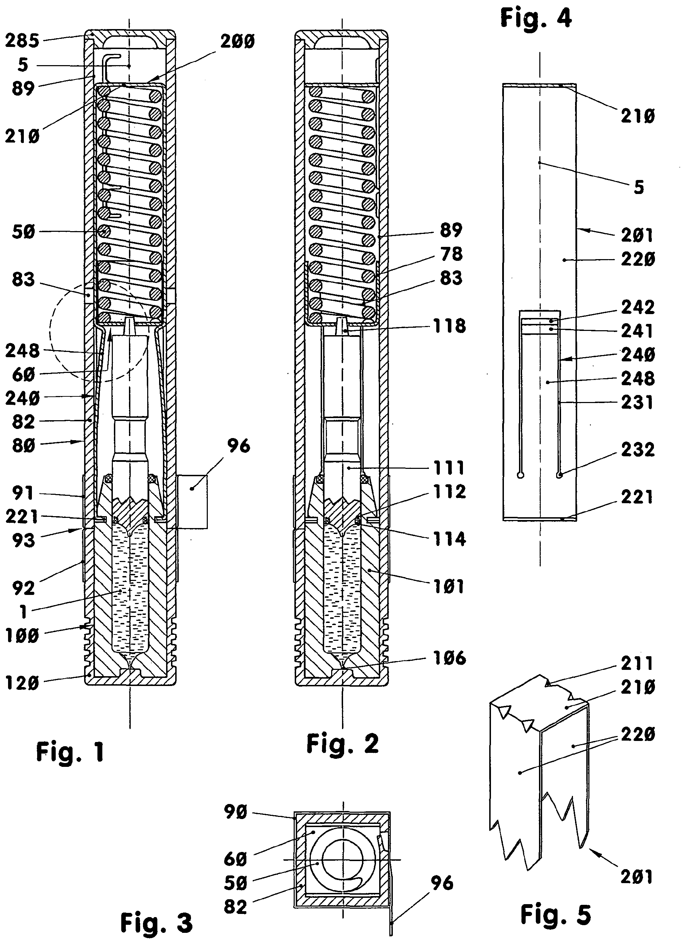

An den Übergangsstellen zwischen der Stirnplatte (210) und den Schenkeln (220) sind nach Figur 5 jeweils zwei Versteifungssicken (211) eingedrückt. Die Versteifungssicken (211) ragen soweit in die Stirnplatte (210) hinein, dass sie zudem die letzte Windung der Schraubendruckfeder (50) auf der Stirnplatte (210) zentrieren.

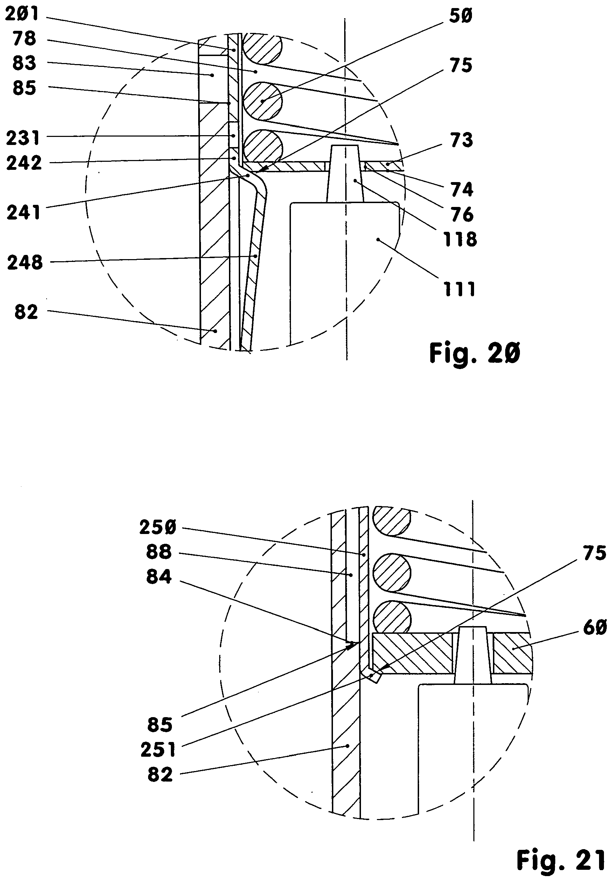

In der unteren Hälfte eines jeden Schenkels (220) ist ein z.B. 9 Millimeter breiter Stützstab (240) angeordnet, vgl. Figur 4. Der Stützstab (240) entsteht durch das Herausarbeiten eines u-förmigen, z.B. 0,2 bis 0,5 Millimeter breiten Spaltes (231). Der Spalt (231) endet im jeweils unteren Bereich der Schenkel (220), also in der Nähe der Halteelemente (221) in Bohrungen (232) zur Minimierung der dortigen Kerbspannungen. Im Gegensatz zu den im Wesentlichen planen Schenkeln (220) ist der Stützstab (240) mehrfach gebogen, vgl. auch Figur 20. Der Stützstab (240) besteht aus einem Biegebalken (248), einem Abstützabschnitt (241) und einem Anlageabschnitt (242). Der Biegebalken (248) leitet die Spannkraft des Federenergiespeichers (50) in den ihn tragenden Schenkel (220) ein. Auf dem Abstützabschnitt (241) liegt der Kolbenbetätigungsstempel (60) bei nicht ausgelöstem Einweginjektor auf. Über den Anlageabschnitt (242) stützt sich der Stützstab (240) am Auslöseelement (82) großflächig ab.

Der Abstützabschnitt (241), er misst in Längsrichtung ca. 1,5 bis 3 Millimeter, vgl. Figur 20, schließt mit dem Biegebalken (248) z.B. einen Winkel von 110 bis 115 Winkelgraden ein. Gegenüber der Vertikalen ist er um 60 Winkelgrade geneigt. Der Anlageabschnitt (242), der in Längsrichtung 1 bis 2 Millimeter breit ist, schließt mit dem Abstützabschnitt (241) einen 140°-Winkel ein. Dabei liegt er nach den Figuren 1 und 4 großflächig am Auslöseelement (82) an. Beispielsweise hat das Auslöseelement (82) im Kontaktbereich eine keramische Panzerung.

Ggf. haben die elastischen Stützstäbe (240) im unteren Bereich eine - zur Mittellinie (5) zumindest annähernd parallele - Längssicke zur Erhöhung ihrer Knicksteifigkeit. Die Stützstäbe (240) federn als elastische Biegebalken (248) immer nach außen, um beim Auslösen die sich längenden Schraubendruckfedern (50) nicht zu bremsen.

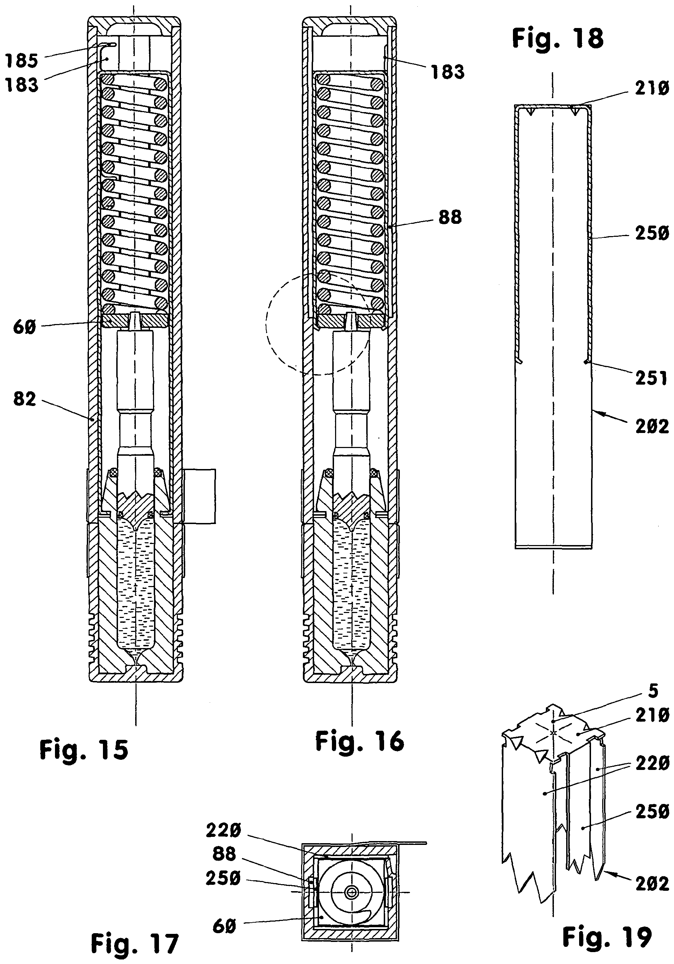

Die Stützstäbe (240) können auch durch Zughaken ersetzt werden. Letztere werden ebenfalls über einen u-förmigen Schlitz gegenüber dem jeweiligen Schenkel (220) abgegrenzt. Allerdings liegen hier dann die Bohrungen (232) in der Nähe der Stirnplatte (210). Der jeweilige Abstützabschnitt der Zughaken ist z.B. so gestaltet, wie die Abstützabschnitte (251) der Zughaken (250) aus Figur 18.

Nach den Figuren 4 und 20 liegt auf den Abstützabschnitten (241) der Stützstäbe (240) der Kolbenbetätigungsstempel (60) auf. Letzterer ist hier ein u-förmig gebogener Blechstreifen, der aus einem Mittelteil, dem Stempelteller (73) und zwei Führungsschenkeln (78) besteht. Der Stempelteller (73) ist parallel zur Stirnplatte (210) orientiert. Die Führungsschenkel (78) stehen rechtwinkelig nach oben ab. Zwischen den Führungsschenkeln (78) sitzt die Schraubendruckfeder (50). Ggf. sind die Führungsschenkel (78) gegenüber dem Stempelteller (73) mit Versteifungssicken versteift, vgl. hierzu die Versteifungssicken (211) des Blechteils (201) aus Figur 5.

Nach Figur 20 hat der Stempelteller (73) in dem Bereich, in dem er an dem jeweiligen Abstützabschnitt (241) des Stützstabs (240) anliegt, z.B. eine 20°-Fase (75) zur Sicherstellung einer großflächigen Anlage.

Gemäß Figur 1 hat der Kolbenbetätigungsstempel (60) eine Breite, die geringfügig - also ca. 0,1 bis 0,3 Millimeter - kleiner ist als der reguläre Abstand der beiden Schenkel (220). Demnach wird der Kolbenbetätigungsstempel (60) seitlich an den Schenkeln (220) geführt. In Figur 2 ist zu erkennen, dass die Führungsschenkel (78) des Kolbenbetätigungsstempels (60) an der Innenwandung (89) des Auslöseelementes (82) mit Spiel geführt anliegen.

Der Stempelteller (73) hat u.a. nach den Figuren 1 bis 3 und 20 eine zentrale Bohrung (76), um den Kolben (111) der Zylinder-Kolben-Einheit (100) rückwärtig zusätzlich zu führen.

Die beiden auf Druck belasteten Stützstäbe (240) halten den Kolbenbetätigungsstempel (60) an dessen Stempelteller (73) in seiner vorgespannten Lage, vgl. Figur 1 und 20. Dazu stützen sich die Stützstäbe (240) mit ihren Abstützabschnitten (241) an der unteren 20°-Fase (75) des Stempeltellers (73) ab. Die Größe der jeweiligen Kontaktfläche zwischen dem einzelnen Abstützabschnitt (241) und der entsprechenden 20°-Fase (75) liegt im Bereich von 5 bis 20 mm².

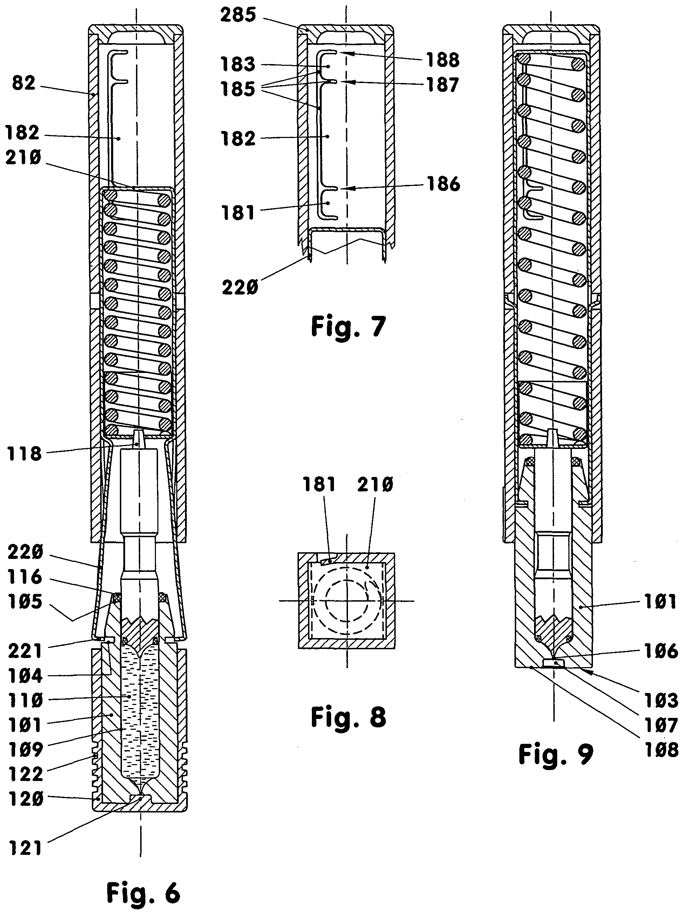

Das aus Blech gefertigte Gehäuse (200) ist großteils von einem Auslöseelement (82) umgeben, in dem es gleitfähig sitzt. Das Auslöseelement (82) ist hier ein mit einem Deckel (285) rückwärtig verschlossenes Vierkantrohr, das Teil einer Auslöseeinheit (80) ist. Das aus Kunststoff, z.B. einem Polyamid gefertigte Vierkantrohr (82), das z.B. eine Wandstärke von 1,5 bis 2,5 Millimeter hat, weist in seinem mittleren Bereich zwei einander gegenüberliegende, z.B. rechteckige Fenster (83) bzw. Durchbrüche auf. Die Fenster (83) sind z.B. 10,5 Millimeter breit und in Längsrichtung, also parallel zur Mittellinie (5), 3,75 Millimeter hoch. Sie nehmen bei einem ausgelösten Injektor jeweils den Anlageabschnitt (242) und den Abstützabschnitt (241) des einzelnen Stützstabes (240) z.B. vollständig auf, vgl. Figur 9.

Im hinteren Bereich des Vierkantrohres (82) sind drei wenige zehntel Millimeter nach innen ragende, elastische Rastlaschen (181-183) angeordnet, vgl. Figur 7 und 8. Die Rastlaschen (181-183) haben z.B. jeweils eine rechteckige Form. Ihre Wandstärke entspricht ca. 50% der Wandstärke des Vierkantrohres (82). Sie grenzen sich an drei Seiten gegenüber der Wandung des Vierkantrohres (82) bzw. gegenüber der nächstgelegenen Rastlasche über Spalte (185) ab. Die Spalte (185) haben eine Breite von z.B. 0,5 Millimeter. Die Breite entspricht der Wandstärke der Stirnplatte (210). An den Stellen, an denen jeweils zwei Spalten (185) rechtwinkelig aneinander stoßen, sind die Rastlaschen (181-183) abgerundet.

Die außermittig angeordneten, am Vierkantrohr angeformten Rastlaschen (181-183) sichern die Position des Blechstreifens (201) an drei Stellen (186-188). Sie ragen dazu mehrere zehntel Millimeter in den Innenraum des Auslöseelements (82) hinein. Die erste Stelle (186) ist der Spalt zwischen der vorderen (181) und der mittleren Rastlasche (182). Im dortigen horizontalen Spalt ist die Stirnplatte (210) eingerastet, vgl. Figur 6, wenn der Blechstreifen (201) mit der zwischen dem Kolbenbetätigungsstempel (60) und der Stirnplatte (210) eingespannten Schraubendruckfeder (50) zur weiteren Zwischenlagerung montiert ist.

Die zweite Stelle (187) ist der Spalt zwischen der mittleren (182) und der hinteren Rastlasche (183). Nach den Figuren 1 und 2 sitzt hier die Stirnplatte (210) bei einem fertig montierten, nicht ausgelösten Einweginjektor. Durch das Einrasten der Stirnplatte (210) in diesen Spalt wird ein Herausziehen des Gehäuses (200) aus dem Vierkantrohr (82) - nach dem Abziehen der Schutzkappe (120) - verhindert. Die dritte Stelle (188) ist der Spalt oberhalb der hinteren Rastlasche (183). In dieser Position verharrt der Blechstreifen (201) nach dem Auslösen des Injektors, vgl. Figur 9. Dort ist er gegen eine unerwünschte Demontage des dann verbrauchten Injektors gesichert.

Ggf. sind die jeweils oberen Ecken der Rastlaschen (181-183) - also die, die dem Deckel (285) zugewandt sind - scharfkantig ausgebildet, so dass der Blechstreifen (201) nur in das Vierkantrohr (82) hineingeschoben werden kann. Eine Bewegung in die entgegengesetzte Richtung ist dann unmöglich.

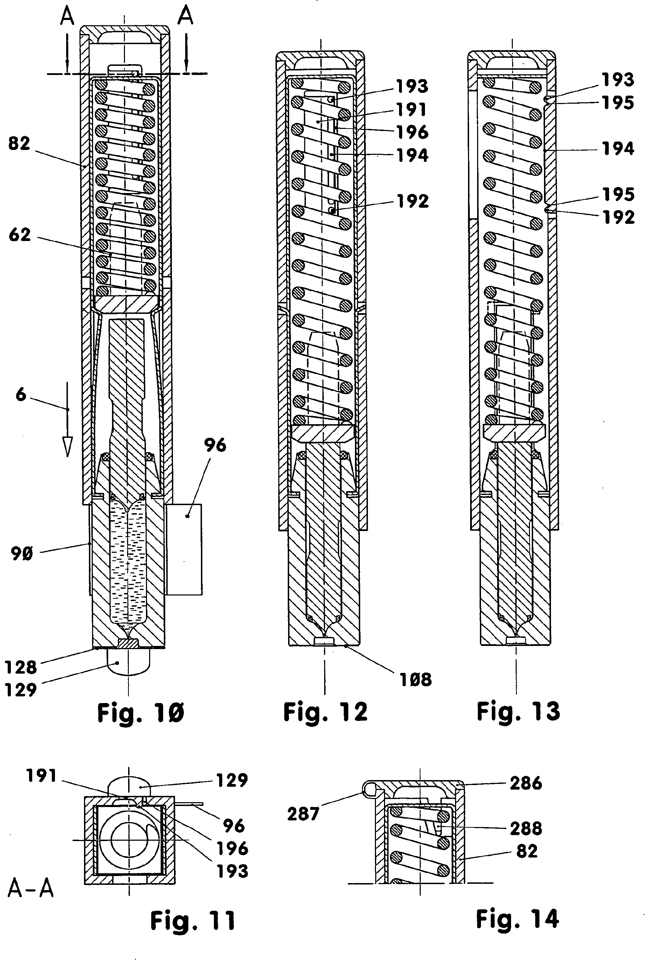

Anstelle der Rastlaschen (181-183) kann auch eine Rastplatte (191) verwendet werden, vgl. Figuren 10 bis 13. Die Rastplatte (191) ist eine dünnwandige, biegeelastische Platte, die im Vierkantrohr (82) integriert ist. Ihre außen liegende Oberfläche schließt bei einer unverformten Rastplatte (191) bündig mit der Außenfläche des Vierkantrohres (82) ab, vgl. Figur 11. Nach innen weist die über einen c-förmigen Spalt (196) freigeschnittene Rastplatte (191) entlang ihrer längeren Innenkante zwei Rastnoppen (192, 193) und einen mittleren Raststeg (194) auf. Zwischen den Rastnoppen (192, 193) und dem Raststeg (194) liegt jeweils eine Kerbe (195), deren Breite im Kerbgrund der Wandstärke des Blechstreifens (201) bzw. der Wandstärke der Stirnplatte (210) entspricht. Die Rastnoppen (192, 193) und der Raststeg (194) ragen bei unverformter Rastplatte (191) in den Innenraum des Vierkantrohres (82) hinein. Der Ort der vorderen Kerbe (195) entspricht als Transportsperre der ersten Stelle (186), vgl. Figur 7, der Blechstreifenparkposition, während der Ort der hinteren Kerbe (195) die zweite Blechstreifenparkposition (187) darstellt, vgl. Figuren 1, 2 und 7.

Die Fenster (83) und die Spalte (185, 196) sind beim fertig montierten Einweginjektor z.B. durch eine dauerhaft aufgeklebte oder aufgeschrumpfte, z.B. beschriftete ggf. elastische Folie staubdicht abgedeckt.

Bei den hier gezeigten Varianten sind alle Rastelemente (181-183; 192-194) am Auslöseelement (82) angeordnet. Sie fixieren teilweise temporär, teilweise dauerhaft die Position der Stirnplatte (210) gegenüber dem Auslöseelement (82). Es ist auch denkbar, die Rastelemente (181-183; 192-194) durch ein am Gehäuse (200) angeordnetes z.B. nockenartiges Rastelement zu ersetzen. Letzteres würde dann in entsprechende Aussparungen des Auslöseelements (82) eingreifen, um vergleichbare Rastpositionen zu realisieren.

Am hinteren Ende ist das Vierkantrohr (82) mit einem Deckel (285) verschlossen. Der Deckel (285) ist beispielsweise mit dem Auslöseelement (82) verklebt, verschweißt, verrastet oder verstaucht. Ggf. ist der Deckel am Auslöseelement (82) auch angeformt.

In Figur 14 ist ein Deckel (285) dargestellt, der am Vierkantrohr (82) über ein Filmgelenk (287) angeformt ist. Im aufgeklappten Zustand wird er zusammen mit dem Vierkantrohr (82) spritzgusstechnisch hergestellt. Vom Deckel (285) steht nach unten ein Rasthaken (288) ab. Der Rasthaken (288) sichert den Blechstreifen (201) in der in Figur 10 dargestellten Position.

Die Zylinder-Kolben-Einheit (100) besteht im Ausführungsbeispiel aus einem, mit einer Injektionslösung (1) oder einem Lösemittel, z.B. Wasser für Injektionszwecke, befüllten, transparenten Zylinder (101), in dem nach Figur 1 ein Kolben (111) in seiner hinteren Position sitzt.

Der Zylinder (101) ist z.B. ein dickwandiger Topf. Die Zylinderbohrung ist beispielsweise zylindrisch oder kegelstumpfmantelförmig ausgeführt. Im Zentrum der Bohrung, deren Zylinderboden der Kontur der vorderen Stirnseite des Kolbens (111) zumindest annähernd angepasst ist, befindet sich z.B. eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101), vgl. Figur 9. Ggf. können im Boden des Zylinders (101) auch zwei oder mehr düsenartige Bohrungen (106) angeordnet sein.

Um die Ausnehmung (107) herum klebt fest haftend auf der Stirnfläche (103) ein Klebering (108). Letzterer deckt nahezu die gesamte Stirnfläche (103) ab.

Die räumliche Außenkontur des Zylinders (101) ist im Ausführungsbeispiel z.B. quaderförmig gestaltet. Sie kann jedoch auch zylindrisch sein. Der Querschnitt der Außenkontur - er ist quer zur Mittellinie (5) orientiert - ist im mittleren Zylinderbereich eine quadratische Fläche mit zentraler Bohrung. Der Querschnitt ist so dimensioniert, dass der Zylinder (101) mit geringem Spiel im Innenraum des Vierkantrohres (82) gleitet.

Der Zylinder (101) hat in seiner Außenkontur im oberen, dem Vierkantrohr (82) zugewandten Viertel eine z.B. umlaufende Haltekerbe (104) mit einem z.B. rechteckigen Kerbquerschnitt. Oberhalb der Haltekerbe (104) verjüngt sich der Zylinder (101) pyramidenstumpfförmig. Der von gegenüberliegenden pyramidalen Flächen eingeschlossene Winkel beträgt z.B. 20 bis 30 Winkelgrade. Die Haltekerbe (104) kann ggf. auch nur aus zwei einander gegenüber liegenden Einzelkerben bestehen.

Der Zylinder (101) hat eine Zylinderinnenwandung (109), die im Bereich der hinteren Zylinderstirnfläche in einer Ringnut (105) zur Aufnahme eines Dichtelements (116) endet.

Der im Zylinder (101) sitzende Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Der Kolben (111) hat in seinem mittleren Bereich eine Taille und an seiner Rückseite z.B. einen zentralen, kegelstumpfförmigen Zapfen (118), der in die Bohrung (76) des Stempeltellers (73) mit Spiel hineinragt.

Der Kolben (111) und das Dichtelement (116) schließen den befüllten Zylinderinnenraum (110) steril ab.

Die zylindrische Ausnehmung (107) der bodenseitigen Stirnfläche (103) des Zylinders (101) ist nach Figur 10 beispielsweise mit einer Schutzfolie (128) verschlossen. Die Schutzfolie (128) klebt über einen Klebering (108) an der Stirnfläche (103). Sie hat seitlich eine Abziehlasche (129). Im mittleren Bereich der Schutzfolie (128) befindet sich ein elastischer an der Schutzfolie (128) fest haftender Stopfen, der den Hohlraum der Ausnehmung (107) dichtend ausfüllt.

Alternativ hierzu ist u.a. in den Figuren 1 und 2 eine topfförmige Schutzkappe (120) von unten her auf den Zylinder (101) aufgesteckt. Die einteilige Schutzkappe (120), die geometrisch im Prinzip aus fünf ebenen Wandungen besteht, umschließt den Zylinder (101) seitlich mit geringem Spiel. Ihre obere, z.B. plane Stirnfläche kontaktiert die vordere Stirnfläche des vierkantförmigen Auslöseelements (82). Die Außenwandung der Schutzkappe (120) weist eine Profilierung oder Struktur auf, um das Abziehen vom Zylinder (101) zu erleichtern. Im Ausführungsbeispiel wird als Profilierung ein Rillenprofil (122) verwendet.

Der Boden der Schutzkappe (120) weist einen Stopfen (121) auf, der dichtend in die Ausnehmung (107) des Zylinders (101) hineinragt. Die Schutzkappe (120) haftet am Zylinder (101) über den Klebering (108). Letzterer hat gegenüber dem Zylinder (101) eine wesentlich höhere Haftkraft als gegenüber dem Boden der Schutzkappe (120). Um die Haftkraftdifferenz zusätzlich sicherzustellen, ist ggf. der Boden mit einem Profil oder Absatz versehen, so dass die Kontaktfläche gegenüber dem Klebering (108) kleiner ist als die Kontaktfläche zwischen dem Klebering (108) und der zylinderseitigen Stirnfläche (103).

Zwischen dem Stempelteller (73) und der Stirnplatte (210) des Blechstreifens (201) sitzt vorgespannt die Schraubendruckfeder (50). Die Federkraft wird über den Stempelteller (73) auf die Stützstäbe (240) übertragen. Aufgrund der Neigung der Fase (75) des Stempeltellers (73) werden die Stützstäbe (240) keilgetriebeartig radial nach außen gedrängt, vgl. Figur 20. Die Fasen (75) kontaktieren die geneigten Abstützabschnitte (241) der Stützstäbe (240). Die Anlageabschnitte (242) liegen zumindest nahezu plan an der Innenwandung des Vierkantrohres (82) an. Das Vierkantrohr (82) stützt somit die keilgetriebebedingte Radialkraft dauerhaft ab.

Nach den Figuren 1 und 2 berühren sich das vierkantförmige Auslöseelement (82) und die Schutzkappe (120) an ihren Stirnseiten. Als Originalitätsverschluss ist dieser Bereich zusätzlich mit einer Banderole (90) als Sicherungselement umgeben. Die abreiß- oder auftrennbare Banderole (90) ist z.B. ein mit einem Klebstoff einseitig beschichteter Papier- oder Folienstreifen. Der Folienstreifen umgibt z.B. einlagig einmal den Verbund aus Auslöseelement (82) und Schutzkappe (120). Er verklebt die Teile (82) und (120) temporär. Zum Entsichern des Injektors bzw. zum Entfernen der Schutzkappe (120) - bei der Vorbereitung zur Nutzung des Injektors - wird die Banderole (90) abgezogen oder so aufgetrennt, dass die Klebeverbindung zwischen dem Auslöseelement (82) und der Schutzkappe (120) aufgehoben ist. Im Ausführungsbeispiel wird dazu die im Bereich des Auslöseelements (82) liegende Abreißfahne (96) ergriffen und damit die Banderole (90) z.B. bereichsweise abgewickelt. Hierbei reißt die Banderole (90) an einer definierten, z.B. geradlinigen Sollbruchstelle (93) auf, die genau im Bereich der Stirnseiten liegt. Folglich wird - beim Entsichern - nur der auf dem Auslöseelement (82) anliegende Teil (91) der Banderole (90) entfernt.

Die Figuren 6 und 7 zeigen den Injektor bei einem Montagezwischenschritt. Bei der Montage wird zunächst die Schraubendruckfeder (50) mit dem Kolbenbetätigungsstempel (60) und dem Blechstreifen (201) zusammengesteckt. Dazu wird die Schraubendruckfeder (50) in den fertig umgeformten Blechstreifen (201) so eingelegt, dass ein Federende an der Stirnplatte (210) zur Anlage kommt. Auf das andere Federende wird der bügelartige Kolbenbetätigungsstempel (60) aufgeschoben. Nun wird unter Zuhilfenahme einer die Schraubendruckfeder (50) außen oder innen führenden Montagevorrichtung der Blechstreifen (201) zwischen der Stirnplatte (210) und dem Kolbenbetätigungsstempel (60) so weit - entgegen der Federwirkung - zusammengeschoben, dass die Fasen (75) der Stirnseite (74) hinter den Abstützabschnitten (241) zur Anlage kommen. Hierbei erleichtern die an dem Kolbenbetätigungsstempel (60) seitlich anliegenden Anlageabschnitte (242) den Montagevorgang.

Nun wird die Kombination aus der gespannten Feder (50), dem Blechstreifen (201) und dem Kolbenbetätigungsstempel (60) - immer noch eingespannt in der Montagevorrichtung - von unten her in das Vierkantrohr (82) eingeschoben. Der Einschiebevorgang ist beendet, wenn die Stirnplatte (210) in den zwischen den Rastlaschen (181) und (182) gelegenen Spalt (185) einrastet. In dieser Position (186), vgl. Figur 6, ragen die freien Enden der Schenkel (220) unten aus dem Vierkantrohr (82) heraus.

In einem weiteren Montageschritt wird die befüllte Zylinder-Kolben-Einheit (100), mit dem Führungszapfen (118) des Kolbens (111) voraus, in das Vierkantrohr (82) so eingesteckt, dass zum einen der Führungszapfen (118) in die Bohrung (76) des Kolbenbetätigungsstempels (60) hineinragt und zum anderen die Halteelemente (221) der Schenkel (220) in die Haltekerbe (104) des Zylinders (101) hineingreifen. Ausgehend von dieser Position wird das Vierkantrohr (82) weiter über den Blechstreifen (201) geschoben, bis die Stirnplatte (210) in den zwischen den Rastlaschen (182) und (183) gelegenen Spalt (185) einrastet. Hierbei greifen die Halteelemente (221) fest in die Haltekerbe (104) ein und fixieren so die Zylinder-Kolben-Einheit (100) im Vierkantrohr (82). Gegenüber dem in Figur 1 dargestellten Montageschritt fehlt nur noch das Anbringen des Originalitätsverschlusses (90) und das Überkleben bzw. Abdecken der Fenster (83) und der Spalte (185, 196) mittels einer beschrifteten Folie.

Die Figuren 10 bis 13 zeigen eine gegenüber den Figuren 1 bis 9 vereinfachte Variante. Sie unterscheidet sich u.a. in sieben Punkten. Erstens haben die Stützstäbe (240) keine separaten Anlageabschnitte, vgl. Figuren 10 und 12 im Gegensatz zu Figur 20. Zweitens ist der Kolbenbetätigungsstempel (60) nur eine Vierkantplatte ohne Bohrung, die an ihrer unteren Stirnseite (74) zwei oder vier Fasen (75) aufweist. Ggf. ist an der oberen Stirnseite der Vierkantplatte ein Führungszapfen (62) - hier gestrichelt eingezeichnet - befestigt oder angeformt. Drittens hat der Kolben (111) an seiner hinteren Stirnseite keinen Führungszapfen. Viertens hat das Auslöseelement (82) anstelle der Rastlaschen (181-183), vgl. Figur 7, eine Rastplatte (191). Fünftens weist der Zylinder (101) anstelle der Schutzkappe (120), vgl. Figur 1, nur eine Schutzfolie (128) auf, vgl. Figur 10 und 11. Sechstens ist die Banderole (90) nur um den Zylinder (101) gewickelt. Allerdings hat die Folie der Banderole (90) eine so große Wandstärke, dass sie ein Verschieben des Auslöseelements (82) in Auslöserichtung (6) sicher blockiert. Siebtens hat das Auslöseelement (82) z.B. einen angeformten Deckel (285) nach Figur 14.

Eine dritte Variante eines Einweginjektors ist in den Figuren 15 bis 19 dargestellt. Dieser Injektor hat als Gehäuse keinen Blechstreifen (201) sondern ein Blechkreuz (202), vgl. Figuren 18 und 19. In Figur 19 ist der obere Bereich des schon umgeformten Blechkreuzes (202) dargestellt. Hier hat das Blechkreuz (202) die schon bekannte Stirnplatte (210), an der zwei breite, lange Schenkel (220) und um 90 Winkelgrade versetzt - geschwenkt um die Mittellinie (5) - zwei schmale kurze Schenkel (250) angeordnet sind. Die genannten Größenverhältnisse sind nur beispielhaft zu verstehen.

Die langen, breiten Schenkel (220) haben die Aufgabe, den Zylinder (101) über die Halteelemente (221) zu halten, vgl. auch Figuren 1 und 2. Allerdings haben bei den Figuren 15 bis 19 diese Schenkel (220) keine Stützstäbe.

Die kurzen, schmalen Schenkel (250) ersetzen als Zughaken die Stützstäbe. Die hier gezeigten Zughaken (250) haben dazu an ihren freien - und nach Figur 18 - unteren Enden jeweils ein Abstützabschnitt (251), der durch ein einfaches nach innen Biegen des Zughakenendes - um z.B. 60 Winkelgrade - entsteht. Auf den Abstützabschnitten (251) liegt bei gespanntem Federenergiespeicher (50) der hier plattenförmige Kolbenbetätigungsstempel (60) mit seinen Fasen (75) auf.

Nach den Figuren 15, 16 und 21 ist der Kolbenbetätigungsstempel (60) eine ebene Platte mit einer z.B. quadratischen oder rechteckigen Stirnfläche, vgl. Figur 17.

Die Zughaken (250) stützen sich bei gespanntem Federenergiespeicher (50) an der Innenwandung des Auslöseelements (82) unterhalb der zu den Längsnuten (88) gehörenden Kanten (85) ab. Auch hier bilden die Abstützabschnitte (251) und die Fasen (75) ein Keilgetriebe, das die Zughaken (250) nach außen drängt. Im ausgebauten Zustand stehen die federelastischen Zughaken (250) nach außen ab. Dadurch weichen sie nach dem Auslösen des Injektors - auch unabhängig von der Wirkung des Keilgetriebes - nach außen hin aus, um die Schraubendruckfeder (50) bei ihrer Längenänderung nicht zu behindern.

Um beim Auslösen des Injektors ein nach außen Drängen der Zughaken (250) zu ermöglichen, benötigt das hier ebenfalls z.B. vierkantförmige Auslöseelement (82) an der Innenwandung (89) die beiden zuvor erwähnten, einander gegenüber liegenden Längsnuten (88), vgl. Figuren 16 und 17. Jede Längsnut (88) endet im mittleren Bereich des Auslöseelements (82) in einer Rücksprungflanke (84), vgl. Figur 21. Beim Auslösen gelangen die Kanten (85) - durch das Verschieben des Auslöseelements (82) - unterhalb die Abstützabschnitte (251) der Zughaken (250), wodurch diese und die Längsnuten (88) zurückweichen.

Wegen der Anordnung der Längsnuten (88) im Vierkantrohr (82) werden die Rastlaschen (181-183), über die das Blechkreuz (202) in verschiedenen Positionen arretiert wird, in Richtung der nächstgelegenen Eckinnenkante verlagert.

Anstelle des beschriebenen gehäuseartigen Blechkreuzes (202) kann auch ein Blechstern mit sechs, acht oder mehr Schenkeln verwendet werden. Beispielsweise hat ein Blechstern eine achteckige Stirnplatte (210), von der zumindest annähernd senkrecht vier Schenkel mit Halteelementen (221) und vier weitere Schenkel mit Abstützabschnitten (251) angeordnet sind. Dabei wechseln sich die Halteelemente (221) und die Abstützabschnitte (251) ab. Die Kolben-Zylinder-Einheit (100) und das Auslöseelement (82) haben hier dann ebenfalls z.B. achteckige Querschnitte. Das Blechkreuz oder der Blechstern kann auch aus einzelnen Blechstreifen gebaut werden, in dem die Blechstreifen im Bereich der Stirnplatte (210) z.B. miteinander verschweißt oder vernietet werden.

Zur Vorbereitung der Benutzung des in den Figuren 1 bis 9 dargestellten Einweginjektors wird dieser zunächst durch das Ablösen der Abreißfahne (96) und des hinteren Banderolenabschnitts (91) entsichert. Anschließend wird die Schutzkappe (120) von der Zylinder-Kolben-Einheit (100) abgezogen. Nun wird der Injektor mit dem Klebering (108) voraus - auf der Injektionsstelle positioniert. Dabei wird der Einweginjektor am Vierkantrohr (82) in der Faust gehalten. Der Daumen der haltenden Hand liegt beispielsweise auf dem Deckel (285) auf, z.B. wie beim Halten eines Kugelschreibers.

Nun wird das Vierkantrohr (82) in Richtung der Zylinder-Kolben-Einheit (100) verschoben. Bei diesem Vorgang gleitet das Auslöseelement (82) auf dem Blechstreifen (201) linear nach unten, also in Richtung der Injektionsstelle. Die Anlageabschnitte (242) der Stützstäbe (240) rutschen über die Kante (85) und springen unter der Kraft des Federelements (50) entsichernd radial nach außen in die Fenster (83). Die Abstützabschnitte (241) geben den Kolbenbetätigungsstempel (60) frei. Dieser schnellt ungehindert nach unten. Dabei schlägt die Stirnseite (74) des Stempeltellers (73) auf die Stirnseite des bisher einige zehntel Millimeter oder wenige Millimeter entfernt gelegenen Kolbens (111). Der Kolben (111) drückt die Injektionslösung bzw. das Medikament (1) z.B. anfangs mit 300 x 10⁵ Pa durch die Düse (106), bis der Zylinder (101) entleert ist, vgl. Figur 9. Mit der Abgabe der Injektionslösung (1) ist der Injektionsvorgang beendet.

Die Ausführungsbeispiele zeigen Injektoren, deren gehäuseseitige Schenkel (220, 250) jeweils paarweise zumindest annähernd parallel - eine Winkelabweichung von ± 2 Winkelgraden ist zulässig - zueinander ausgerichtet sind. Die Schenkel (220, 250) liegen hierbei in parallelen Ebenen, wobei die Ebenen - im Injektorquerschnitt gesehen - die einander gegenüberliegenden Seiten eines Rechtecks bilden. Die Ebene des Injektorquerschnitts liegt normal - also senkrecht - zur Mittellinie (5). Diese Seiten können auch zu einer Raute, einem Parallelogramm, einem Trapez oder einem schiefwinkeligen Viereck gehören.

Zudem sind die Schenkel (220, 250) und die Druckstäbe (240) jeweils paarweise gleich lang. Das ist nicht zwingend notwendig. So können z.B. die Druckstäbe (240) unterschiedlich lang sein, wenn entsprechend die Auflageflächen des Kolbenbetätigungsstempels (60) und die Fenster (83) versetzt positioniert werden.

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil

- 50: Federelement, Schraubendruckfeder, Federenergiespeicher

- 60: Kolbenbetätigungsstempel
- 62: Führungszapfen

- 73: Stempelteller
- 74: Stirnseite, unten
- 75: Fase, 20°-Fase
- 76: Bohrung
- 78: Führungsschenkel

- 80: Auslöseeinheit
- 82: Auslöseelement, Vierkantrohr
- 83: Fenster, Durchbrüche
- 84: Rücksprungflanke
- 85: Kante, scharfkantig
- 88: Längsnuten
- 89: Innenwandung

- 90: Originalitätsverschluss, Banderole, Sicherungselement
- 91: hinterer Banderolenabschnitt, an (82); Teil
- 92: vorderer Banderolenabschnitt, an (120)
- 93: Sollbruchstelle, Perforation
- 96: Abreißfahne

- 100: Zylinder-Kolben-Einheit
- 101: Zylinder
- 103: Stirnfläche
- 104: Haltekerbe
- 105: Ringnut
- 106: Bohrung, Düse
- 107: Ausnehmung in der Stirnfläche
- 108: Klebering
- 109: Zylinderinnenwandung
- 110: Zylinderinnenraum
- 111: Kolben
- 112: Ringnut
- 114: Dichtring, Dichtung
- 116: Dichtelement in (105)
- 118: Führungszapfen
- 120: Schutzkappe
- 121: Stopfen
- 122: Rillenprofil
- 128: Schutzfolie, Klebeversiegelung
- 129: Abziehlasche

- 181: Rastlasche, vorn; Rastelement
- 182: Rastlasche, Mitte; Rastelement
- 183: Rastlasche, hinten; Rastelement

- 185: Spalte
- 186: 1. Stelle
- 187: 2. Stelle
- 188: 3. Stelle

- 191: Rastplatte
- 192, 193: Rastnoppen; Rastelement
- 194: Raststeg; Rastelement
- 195: Kerben
- 196: Spalt, c-förmig

- 200: Gehäuse; Blechteil, dünnwandig
- 201: Blechstreifen; Blechteil
- 202: Blechkreuz; Blechteil

- 210: Stirnplatte
- 211: Sicken, Versteifungssicken

- 220: Schenkel, lang und breit
- 221: Halteelemente

- 231: Spalt, u-förmig
- 232: Bohrungen

- 240: Stützstäbe, Druckstäbe
- 241: Abstützabschnitt
- 242: Anlageabschnitt
- 248: Biegebalken

- 250: Zughaken; Schenkel, kurz und schmal
- 251: Abstützabschnitt

- 285: Deckel
- 286: Deckel mit Filmgelenk
- 287: Filmgelenk
- 288: Rasthaken

## Patentansprüche

1. Nadelloser Einmalinjektor mit einem Gehäuse (200), in dem oder an dem - jeweils zumindest bereichsweise - mindestens ein mechanischer Federenergiespeicher (50),
mindestens eine - zumindest zeitweise wirkstoffbefüllbare - Zylinder-Kolben-Einheit (100),
mindestens ein Kolbenbetätigungsstempel (60) und mindestens eine Auslöseeinheit (80) angeordnet ist,
- wobei der Kolbenbetätigungsstempel (60) zwischen dem Federenergiespeicher (50) und dem Kolben (111) der Zylinder-Kolben-Einheit (100) positioniert ist,
- wobei der Federenergiespeicher mindestens ein vorgespanntes Federelement (50) umfasst und
- wobei der federbelastete Kolbenbetätigungsstempel (60) über Stützstäbe (240) oder Zughaken (250) am Gehäuse (200) abgestützt ist, wobei die zwischen einem einzelnen Stützstab (240) oder Zughaken (250) und dem Kolbenbetätigungsstempel (60) gelegene Kontaktzone ein den jeweiligen Stützstab (240) oder Zughaken (250) nach außen drängendes Keilgetriebepaar darstellt,
**dadurch gekennzeichnet,**
- **dass** das Gehäuse (200) aus einem dünnwandigen Blechteil (201, 202) besteht,
- **dass** das Blechteil (201, 202) eine zentrale Stirnplatte (210) und mindestens zwei davon annähernd senkrecht abstehende Schenkel (220, 250) aufweist,
- **dass** die Schenkel (220) an den freien Enden - als Aufnahme des Zylinders (101) der Zylinder-Kolben-Einheit (100) - jeweils ein abgewinkeltes Halteelement (221) aufweisen, die in eine Haltekerbe (104) der Außenkontur des Zylinders (101) eingreifen,
- **dass** das Blechteil (201, 202) mindestens zwei Druckstäbe (240) oder mindestens zwei Zugstäbe (250) aufweist, deren freie Enden zur Ausbildung eines Abstützabschnitts (241, 251) für den Kolbenbetätigungsstempel (60) jeweils winkelförmig abgebogen sind,
- **dass** die Auslöseeinheit (80) mindestens ein auf dem Gehäuse (200) gleitfähig angeordnetes Auslöseelement (82) umfasst,
- wobei das Auslöselement (82) ein das Blechteil (201, 202) umgebendes Vierkantrohr ist,
- wobei das Auslöselement (80) die den Kolbenbetätigungsstempel (60) in seiner vorgespannten Lage haltenden Stützstäbe (240) oder Zughaken (250) in einer Sperrposition hält und zum Auslösen des Einweginjektors in Längsrichtung (5) beweglich in eine Position gebracht wird, in der das durch das Keilgetriebepaar verursachte radiale nach außen Drängen der Stützstäbe (240) oder der Zughaken (250) freigegeben wird,
und das Auslöseelement (82) mit Fenstern (83) ausgebildet ist, die die Abstützabschnitte (241) der Stützstäbe (240) nach dem Auslösen des Einmalinjektors aufnehmen, oder mit Längsnuten (88) ausgebildet ist, die die Abstützabschnitte (251) der Zughaken (250) nach dem Auslösen des Einmalinjektors aufnehmen.

2. Einmalinjektor gemäß Anspruch 1,
**dadurch gekennzeichnet,**
- **dass** das Gehäuse (200) aus einem Blechstreifen (201) besteht,
- **dass** der Blechstreifen (201) zur Ausbildung zweier Schenkel (220) u-förmig gebogen ist,
- **dass** der Blechstreifen (201) an den beiden freien Enden - als Auflage für den Kolbenbetätigungsstempel (60) - nach innen abgewinkelte Halteelemente (221) aufweist,
- **dass** in mindestens einem Schenkel (220) ein Stützstab (240) oder ein Zughaken (250) eingearbeitet ist, der an seinem freien Ende, zur Ausbildung eines Abstützabschnitts (241, 251) für den Kolbenbetätigungsstempel (60), winkelförmig abgebogen ist.

3. Einmalinjektor gemäß Anspruch 1,
**dadurch gekennzeichnet,**
**dass** das Blechteil (201, 202) aus einem Federstahl gefertigt ist.

4. Einmalinjektor gemäß Anspruch 1,
**dadurch gekennzeichnet,**
**dass** das Auslöseelement (82) an mindestens zwei Stellen (186, 187) Rastkerben zur temporären Sicherung der Position des Blechteils (201, 202) hat.

5. Einmalinjektor gemäß Anspruch 1,
**dadurch gekennzeichnet,**
**dass** der Kolbenbetätigungsstempel (60) eine ebene Platte (73) mit rechteckiger Grundfläche ist oder aus einem u-förmig gebogenen Blechstreifen (73, 78) besteht.

6. Einmalinjektor gemäß Anspruch 1,
**dadurch gekennzeichnet,**
**dass** die Platte (73) oder der Blechstreifen (73, 78) in seinem quer zur Mittellinie (5) des Injektors gelegenen Bereich eine zentrale Bohrung (76) aufweist.

7. Einmalinjektor gemäß Anspruch 1,
**dadurch gekennzeichnet,**
**dass** der Kolben (111) der Zylinder-Kolben-Einheit (100) rückseitig einen Führungszapfen (118) aufweist.

8. Einmalinjektor gemäß Anspruch 1,
**dadurch gekennzeichnet,**
**dass** der Kolbenbetätigungsstempel (60) zusammen mit jedem einzelnen Stützstab (240) ein Schiebekeilgetriebe bildet, in dem eine axiale Federkraftrichtung in eine radiale Stützkraftrichtung umgelenkt wird.

9. Einmalinjektor gemäß Anspruch 1,
**dadurch gekennzeichnet,**
**dass** der einzelne Stützstab (240) jeweils am Gehäuse (200) angeformt ist und einen elastischen Biegebalken (248) darstellt.

10. Einmalinjektor gemäß Anspruch 1,
**dadurch gekennzeichnet,**
**dass** das Auslöseelement (82) in Kombination mit dem Gehäuse (200) und einer an ihm befestigten Abreißbanderole (90) eine Auslöseinheit (80) bildet.

## Claims

1. Needleless disposable injector with a housing (200) in which or on which are arranged, in each case at least in some areas, at least one mechanical spring energy reservoir (50),
at least one cylinder/piston unit (100) that can be filled at least temporarily with active substance,
at least one piston-actuating ram (60) and at least one trigger unit (80),
- wherein the piston-actuating ram (60) is positioned between the spring energy reservoir (50) and the piston (111) of the cylinder/ piston unit (100),
- wherein the spring energy reservoir comprises at least one pretensioned spring element (50), and
- wherein the spring-loaded piston-actuating ram (60) is supported on the housing (200) via support rods (240) or tension hooks (250), and wherein the contact zone situated between an individual support rod (240) or tension hook (250) and the piston-actuating ram (60) represents a wedge gear pairing that forces the respective support rod (240) or tension hook (250) outwards,
**characterized in that**
- the housing (200) is made from a thin-walled sheet-metal part (201, 202),
- the sheet-metal part (201, 202) has a central end plate (210) and at least two branches (220, 250) projecting therefrom virtually perpendicularly,
- the branches (220) each have, at the free ends thereof, an angled retaining element (221) as a means of receiving the cylinder (101) of the cylinder/piston unit (100), which angled retaining elements engage in a holding notch (104) of the outer contour of the cylinder (101),
- the sheet-metal part (201, 202) has at least two pressure rods (240) or at least two tension hooks (250), the free ends of which are each bent at an angle to form a supporting portion (241, 251) for the piston-actuating ram (60),
- the trigger unit (80) comprises at least one trigger element (82) arranged slidably on the housing (200),
- wherein the trigger element (82) is a square tube surrounding the sheet-metal part (201, 202),
- wherein the trigger element (80) holds the support rods (240) or tension hooks (250), which hold the piston-actuating ram (60) in its pretensioned position, in a blocking position and, in order to trigger the disposable injector, is brought movably in the longitudinal direction (5) to a position in which the radially outward forcing of the support rods (240) or of the tension hooks (250) caused by the wedge gear pairing is released,
and the trigger element (82) is configured with windows (83), which receive the supporting portions (241) of the support rods (240) after the triggering of the disposable injector, or is configured with longitudinal grooves (88) which receive the supporting portions (251) of the tension hooks (250) after the triggering of the disposable injector.

2. Disposable injector according to Claim 1, **characterized in that**
- the housing (200) is made from a sheet-metal strip (201),
- the sheet-metal strip (201) is bent in a U-shape to form two branches (220),
- the sheet-metal strip (201) has, at both free ends, inwardly angled retaining elements (221) as a bearing for the piston-actuating ram (60),
- a support rod (240) or a tension hook (250) is worked into at least one branch (220) and is bent at an angle, at its free end, to form a supporting portion (241, 251) for the piston-actuating ram (60).

3. Disposable injector according to Claim 1, **characterized in that**
the sheet-metal part (201, 202) is made from a spring steel.

4. Disposable injector according to Claim 1, **characterized in that**
the trigger element (82) has, at least at two locations (186, 187), locking notches for temporarily securing the position of the sheet-metal part (201, 202).

5. Disposable injector according to Claim 1, **characterized in that**
the piston-actuating ram (60) is a flat plate (73) with a rectangular surface area or is made from a sheet-metal strip (73, 78) bent in a U-shape.

6. Disposable injector according to Claim 1, **characterized in that**
the plate (73) or the sheet-metal strip (73, 78) has a central bore (76) in its area situated transverse to the centre line (5) of the injector.

7. Disposable injector according to Claim 1, **characterized in that**
the piston (111) of the cylinder/piston unit (100) has a guide pin (118) on its rear face.

8. Disposable injector according to Claim 1, **characterized in that**
the piston-actuating ram (60), together with each individual support rod (240), forms a spline gear in which an axial spring force direction is converted into a radial supporting force direction.

9. Disposable injector according to Claim 1, **characterized in that**
the individual support rod (240) is in each case formed integrally on the housing (200) and represents an elastic flexural beam (248).

10. Disposable injector according to Claim 1, **characterized in that**
the trigger element (82), in combination with the housing (200) and with a tear-off banderole (90) secured thereon, forms a trigger unit (80).

## Revendications

1. Injecteur à usage unique sans aiguille, comprenant un boîtier (200) dans lequel ou sur lequel sont disposés - à chaque fois au moins en partie - au moins un accumulateur d'énergie à ressort mécanique (50),
au moins une unité cylindre-piston (100), pouvant être remplie de substance active au moins temporairement,
au moins un poinçon d'actionnement de piston (60) et au moins une unité de déclenchement (80),
- le poinçon d'actionnement de piston (60) étant positionné entre l'accumulateur d'énergie à ressort (50) et le piston (111) de l'unité cylindre-piston (100),
- l'accumulateur d'énergie à ressort comprenant au moins un élément de ressort précontraint (50) et
- le poinçon d'actionnement de piston (60) sollicité par ressort étant supporté par le biais de barres de support (240) ou de crochets de traction (250) sur le boîtier (200), la zone de contact située entre une barre de support individuelle (240) ou un crochet de traction individuel (250) et le poinçon d'actionnement de piston (60) constituant une paire de mécanismes à clavette repoussant vers l'extérieur la barre de support (240) ou le crochet de traction (250) respectif, **caractérisé en ce que**
- le boîtier (200) se compose d'une pièce en tôle à paroi mince (201, 202),
- la pièce en tôle (201, 202) présente une plaque frontale centrale (210) et au moins deux branches (220, 250) faisant saillie depuis celle-ci approximativement perpendiculairement,
- les branches (220) présentent, au niveau des extrémités libres - en tant que logement du cylindre (101) de l'unité cylindre-piston (100) - à chaque fois un élément de retenue coudé (221), lesquels éléments de retenue s'engagent dans une encoche de retenue (104) du contour extérieur du cylindre (101),
- la pièce en tôle (201, 202) présente au moins deux barres de pression (240) ou au moins deux barres de traction (250) dont les extrémités libres sont à chaque fois cintrées sous forme coudée pour réaliser une portion de support (241, 251) pour le poinçon d'actionnement de piston (60),
- l'unité de déclenchement (80) comprend au moins un élément de déclenchement (82) disposé de manière à pouvoir coulisser sur le boîtier (200),
- l'élément de déclenchement (82) étant un tube carré entourant la pièce en tôle (201, 202),
- l'élément de déclenchement (80) retenant dans une position de blocage les barres de support (240) ou les crochets de traction (250) retenant le poinçon d'actionnement de piston (60) dans sa position précontrainte et, pour le déclenchement de l'injecteur à usage unique, étant amené de manière déplaçable dans une direction longitudinale (5) dans une position dans laquelle le repoussement radial vers l'extérieur des barres de support (240) ou des crochets de traction (250), provoqué par la paire de mécanismes à clavette, est libéré,
et l'élément de déclenchement (82) étant réalisé avec des fenêtres (83) qui reçoivent les portions de support (241) des barres de support (240) après le déclenchement de l'injecteur à usage unique, ou étant réalisé avec des rainures longitudinales (88) qui reçoivent les portions de support (251) des crochets de traction (250) après le déclenchement de l'injecteur à usage unique.

2. Injecteur à usage unique selon la revendication 1, **caractérisé en ce que**
- le boîtier (200) se compose d'une bande de tôle (201),
- la bande de tôle (201) est cintrée en forme de U pour réaliser deux branches (220),
- la bande de tôle (201) présente au niveau des deux extrémités libres - en tant qu'appui pour le poinçon d'actionnement de piston (60) - des éléments de retenue coudés vers l'intérieur (221),
- une barre de support (240) ou un crochet de traction (250) est incorporé(e) dans au moins une branche (220), laquelle ou lequel est cintré(e) sous forme coudée au niveau de son extrémité libre pour constituer une portion de support (241, 251) pour le poinçon d'actionnement de piston (60).

3. Injecteur à usage unique selon la revendication 1, **caractérisé en ce que**
la pièce en tôle (201, 202) est fabriquée à partir d'un acier à ressort.

4. Injecteur à usage unique selon la revendication 1, **caractérisé en ce que**
l'élément de déclenchement (82) présente en au moins deux endroits (186, 187) des encoches d'encliquetage pour la fixation temporaire de la position de la pièce en tôle (201, 202).

5. Injecteur à usage unique selon la revendication 1, **caractérisé en ce que**
le poinçon d'actionnement de piston (60) est une plaque plane (73) avec une surface de base rectangulaire ou se compose d'une bande de tôle (73, 78) cintrée en forme de U.

6. Injecteur à usage unique selon la revendication 1, **caractérisé en ce que**
la plaque (73) ou la bande de tôle (73, 78) présente, dans sa région située transversalement par rapport à l'axe médian (5) de l'injecteur, un alésage central (76).

7. Injecteur à usage unique selon la revendication 1, **caractérisé en ce que**
le piston (111) de l'unité cylindre-piston (100) présente du côté arrière un tourillon de guidage (118).

8. Injecteur à usage unique selon la revendication 1, **caractérisé en ce que**
le poinçon d'actionnement de piston (60) forme conjointement avec chaque barre de support individuelle (240) un mécanisme à clavette coulissant dans lequel une direction de force de ressort axiale est déviée dans une direction de force de support radiale.

9. Injecteur à usage unique selon la revendication 1, **caractérisé en ce que**
la barre de support individuelle (240) est façonnée à chaque fois sur le boîtier (200) et constitue une poutre flexible élastique (248).

10. Injecteur à usage unique selon la revendication 1, **caractérisé en ce que**
l'élément de déclenchement (82), conjointement avec le boîtier (200) et une banderole d'usure (90) fixée sur celui-ci, forme une unité de déclenchement (80).
